# EUROPEAN PATENT APPLICATION

(11) **EP 4 793 282 A1**
(43) Date of publication of application: **19.08.2026**
(21) Application number: 24876659.4
(22) Date of filing: 11.10.2024
(51) Int. Cl.: C07K 14/47, A61K 45/00, A61P 35/00, C12Q 1/6886, C12N 15/12

(54) **NOVEL MICROPEPTIDE MP36 AND USE THEREOF**

(30) Priority: 13.10.2023 CN 202311328332
(71) Applicant: Nanjing Anji Biotechnology Co., Ltd., Nanjing, Jiangsu 210033 (CN)
(72) Inventor: XU, Hanmei, Nanjing, Jiangsu 211100 (CN)
(74) Representative: Alpspitz IP
(86) International application number: PCT/CN2024/124286
(87) International publication number: WO 2025/077854

(57) **Abstract**

Provided are a novel micropeptide MP36 and a use thereof. The micropeptide MP36 can be used for preparing reagents or drugs for detecting, preventing or treating tumors, wherein the tumors comprise one or more of lung cancer, breast cancer, colon cancer, glioma, bladder cancer, gastric cancer, head and neck cancer, and sarcoma. In tumors, the micropeptide MP36 can inhibit tumor cell growth and/or metastasis by inhibiting MP36 micropeptide expression by means of specific small interfering RNA, or inhibit tumor cell growth and/or metastasis by overexpressing MP36, or inhibit tumor growth and/or metastasis by coupling cell-penetrating peptides, and has important new drug development value and tumor detection value.

## Description

### TECHNICAL FIELD

The present invention pertains to the technical field of biomedicine and particularly relates to a micropeptide MP36 with a novel structure and a use thereof.

### BACKGROUND ART

Long non-coding RNA (lncRNA) is an RNA molecule with a transcript length exceeding 200nt. LncRNAs lack specific complete open reading frames (ORFs), have no protein-coding function and account for a considerable proportion of the total non-coding RNAs (ncRNAs). Initially, lncRNAs were considered as "noise" in genomic transcription and having no biological functions. However, the studies in recent years have shown that lncRNAs regulate gene expression levels in multiple aspects (such as epigenetic regulation, transcriptional regulation, and post-transcriptional regulation) in the form of RNA. Nevertheless, it is still widely believed that lncRNAs do not code any proteins. In 2011, American scientist Jonathan S. Weissman discovered through the ribosome display technology that there were a large number of lincRNAs bound to ribosomes, indicating that they might be translated. In 2015, Eric Olson and his colleagues from the University of Texas Southwestern Medical Center discovered a lncRNA that was specifically expressed in skeletal muscle, proving that this lncRNA coded a micropeptide comprising 46 amino acids in vivo. They named this lncRNA myoregulin. In the recent two years, not many reports have been published on lncRNA being capable of coding micropeptides in the world. The research has focused on muscle differentiation and skeletal muscle development. Therefore, discovering and identifying new micropeptides coded by lncRNAs, and exploring new application fields are of great significance for opening the coding door of lncRNAs.

LncRNA CSORF66-AS1, also known as Epist, is located on human chromosome 5. The research reports that the expression of C5ORF66-AS1 is significantly downregulated in human esophageal squamous cell carcinoma and pituitary adenoma, and it can effectively inhibit the malignant progression and invasion of tumors. However, there is no report yet on whether CSORF66-AS1 plays a role in other types of malignant tumors, or whether it is related to other diseases. Malignant tumors are the leading cause of human health problems. Their uncontrolled growth, invasion and metastasis are the hallmarks of malignancy and are the main reasons for treatment failure and death. At present, one of the main treatment methods for cancer patients in clinical practice remains the drug therapy. However, due to the tendency of tumors to metastasize and the severe heterogeneity of tumor cells, the therapeutic effect of drugs is severely limited. People are constantly exploring and researching the causes of tumors, summarizing treatment and prevention strategies for tumors, and developing new and effective drugs to treat tumors. These are the key to clinical treatment of tumors.

### SUMMARY OF THE INVENTION

The first objective of the present invention is to provide a novel micropeptide MP36, which is a polypeptide with a novel structure that has been discovered for the first time.

To achieve the foregoing technical objective, the present invention adopts the following technical solution:
A micropeptide, wherein the amino acid sequence of the micropeptide is any of the following ones:
(a) an amino acid sequence shown in SEQ ID NO. 1;
(b) an amino acid sequence that has a homology of more than 85% with the amino acid sequence shown in SEQ ID NO. 1;
(c) an amino acid sequence formed by replacing, deleting or adding one or several amino acid residues in the amino acid sequence shown in SEQ ID NO.1, which has the same function as the original sequence.

The second objective of the present invention is to provide a nucleotide sequence coding the foregoing micropeptide.

The third objective of the present invention is to provide a recombinant vector containing the foregoing nucleotide sequence.

The fourth objective of the present invention is to provide a use of the foregoing micropeptide in preparing reagents or drugs for detecting, preventing or treating tumors. The micropeptide is used in the form of amino acids or nucleotides.

As a preferred embodiment, the tumors are one or more of human lung cancer, breast cancer, colon cancer, glioma, bladder cancer, gastric cancer, head and neck cancer, and sarcoma.

As a preferred embodiment, the treatment of tumors is achieved by overexpressing the nucleotide sequence shown in SEQ ID NO. 2 in the tumors; or by using the specific small interfering RNA of the nucleotide sequence shown in SEQ ID NO. 2; or by coupling the micropeptide with cell-penetrating peptides. Specifically, for breast cancer and lung cancer, the treatment of tumors is achieved by using the specific small interfering RNA of the nucleotide sequence shown in SEQ ID NO.2 to inhibit the expression of MP36; for colon cancer, glioma, bladder cancer, gastric cancer, head and neck cancer, and sarcoma tissues, the treatment of tumors is achieved by overexpressing the nucleotide sequence shown in SEQ ID NO. 2 in the tumors.

As a preferred embodiment, the nucleotide sequence of the specific small interfering RNA is shown in SEQ ID NO: 5.

As a preferred embodiment, the prevention or treatment of tumors includes inhibiting the growth and/or metastasis of tumor cells.

As a preferred embodiment, the tumor cells include: tumor cells of head and neck cancer: CAL27 and/or KB; tumor cells of colon cancer: HT29 and/or SW480; tumor cells of glioma: U87-MG; tumor cells of bladder cancer: T24 and/or EJ; tumor cells of gastric cancer: MGC-803 and/or BGC-823; tumor cells of sarcoma: MG63 and/or HT-1080; tumor cells of breast cancer: MDA-MB-231 and/or MCF7; tumor cells of lung cancer: A459.

The fifth objective of the present invention is to provide a pharmaceutical composition. The pharmaceutical composition contains the foregoing micropeptide, or specific small interfering RNA of the micropeptide, or a molecule that promotes the degradation of the micropeptide and a pharmaceutically acceptable carrier thereof. Specifically, for breast cancer and lung cancer, by using a pharmaceutical composition containing specific small interfering RNA of micropeptide and a pharmaceutically acceptable carrier thereof to inhibit the expression of MP36, or by using a pharmaceutical composition containing a molecule that promotes the degradation of the micropeptide and a pharmaceutically acceptable carrier thereof to degrade MP36 to lower its expression, prevention or treatment of tumors is achieved; for colon cancer, glioma, bladder cancer, gastric cancer, head and neck cancer and sarcoma tissues, by means of a pharmaceutical composition containing micropeptide and a pharmaceutically acceptable carrier thereof, prevention or treatment of tumors is achieved. When the pharmaceutical composition contains micropeptides, the micropeptides can exist in the form of free peptide, overexpressed vectors containing micropeptide genes, and so on.

The sixth objective of the present invention is to provide a tumor detection kit. The kit contains specific primer pairs and/or antibodies designed for the foregoing nucleotide sequence.

As a preferred embodiment, the nucleotide sequences of the specific primer pairs are shown in SEQ ID NO. 3 and SEQ ID NO. 4.

Compared with the prior art, the present invention has the following advantages:
(1) The present invention has for the first time discovered that lncRNA C5ORF66-AS1 has a coding ability, and is able to translate a micropeptide with a length of 36 amino acids (named MP36);
(2) The present invention, through data analysis of expression profiling chips, discovered that the expression levels of MP36 in breast cancer and lung cancer were significantly higher than that in normal tissues, while its expression levels in colon cancer, glioma, bladder cancer, gastric cancer, head and neck cancer, and sarcoma tissues were significantly lower than that in normal tissues. This indicates that MP36 can serve as a new tumor marker for assistant diagnosis of tumors in the early stage;
(3) The present invention detected the RNA expression levels of MP36 in head and neck cancer or breast cancer samples, as well as the corresponding adjacent tissues by designing specific primers for the nucleotide sequence coding MP36, and using the fluorescence quantitative PCR method, and discovered that MP36 was significantly underexpressed in head and neck cancer tissues and significantly overexpressed in breast cancer tissues, suggesting that the specific primers designed for the MP36 sequence can be used for tumor diagnosis;
(4) By preparing a specific antibody for MP36 and detecting the level of MP36 in breast cancer and adjacent samples by the immunohistochemical method, the present invention discovered that MP36 was significantly overexpressed in breast cancer tissues, suggesting that the antibody for MP36 can be used for tumor diagnosis;
(5) Through extensive experiments, the present invention discovered that the endogenous overexpression of micropeptide MP36 could significantly inhibit the growth and/or metastasis of head and neck cancer CAL27 cells;
(6) By designing specific siRNAs targeting the nucleotide sequence coding micropeptide MP36, the present invention discovered that it could significantly inhibit the growth and/or metastasis of breast cancer cells MDA-MB-231 and MCF7, as well as the growth of lung cancer cells A549.
(7) By coupling TAT cell-penetrating peptides with the MP36 micropeptide sequence, the present invention discovered that it could significantly inhibit the growth and/or metastasis of head and neck cancer CAL27 and/or KB cells, colon cancer HT29 and/or SW480 cells, glioma U87-MG cells, bladder cancer T24 and/or EJ cells, gastric cancer MGC-803 and/or BGC-823 cells, sarcoma MG63 and/or HT-1080 cells.
(8) The experiments designed by the present invention are scientific, reasonable, feasible and effective. Based on the above findings, the expression level of MP36 can be used as a new biomarker to assist in the diagnosis of various malignant tumors, including lung cancer, breast cancer, colon cancer, glioma, bladder cancer, gastric cancer, head and neck cancer, and sarcoma. The micropeptide MP36 can directly serve as a therapeutic drug for malignant tumors or as a target for anti-tumor drugs, significantly expanding the treatment spectrum of this micropeptide and providing new ideas and prospects for future drug development.

### BRIEF DESCRIPTION OF THE DRAWINGS

FIG. 1 shows the expression level of the target band of MP36 detected by Western blot.
FIG. 2 shows the comparison of the expression levels of C5ORF66-AS1 in human tumor tissues and normal tissues. The data are from the TCGA database.
FIG 3 shows the expression of micropeptide MP36 in human head and neck cancer tissues and adjacent tissues as detected by fluorescence quantitative PCR.
FIG 4 shows the expression of micropeptide MP36 in human breast cancer tissues and adjacent tissues as detected by immunohistochemistry.
FIG. 5 shows the inhibitory effect of micropeptide MP36 on the proliferation of human head and neck cancer CAL27 cells.
FIG. 6 shows the inhibitory effect of micropeptide MP36 on the migration of human head and neck cancer CAL27 cells.
FIG. 7 shows the inhibitory effect of micropeptide MP36 siRNA on the proliferation of human breast cancer MDA-MB-231 cells.
FIG. 8 shows the inhibitory effect of micropeptide MP36 siRNA on the proliferation of human breast cancer MCF7 cells.
FIG. 9 shows the inhibitory effect of micropeptide MP36 siRNA on the proliferation of human lung cancer A549 cells.
FIG. 10 shows the inhibitory effect of micropeptide MP36 siRNA on the migration of human breast cancer MDA-MB-231 cells.
FIG. 11 shows the inhibitory effect of micropeptide MP36 siRNA on the migration of human breast cancer MCF7 cells.
FIG. 12 shows the effect of micropeptide MP36 siRNA on the migration of human lung cancer A549 cells.
FIG. 13 shows the effect of micropeptide MP36 and its homologous mutants on the proliferation of human breast cancer MDA-MB-231 cells.
FIG. 14 shows the effect of micropeptide MP36 and its homologous mutants on the migration of human breast cancer MDA-MB-231 cells.

### DETAILED DESCRIPTION

The present invention will be further described below in conjunction with embodiments.

### Embodiment 1

Confirmation of the in vivo coding ability of C5ORF66-AS1.

Precisely insert a Flag tag in front of the termination codon of the sORF contained in CSORF66-AS1 by applying the CRISPR/cas9 gene editing technology, amplify and collect the cells after determining the correctly inserted target cells by gene sequencing, centrifuge and then discard the supernatant, wash twice with PBS and discard the supernatant. Add an RIPA lysis buffer and perform lysis on ice for 20 min. Centrifuge at 12,000g for 10 min and collect the supernatant. Add a 1XSDS loading buffer, mix well by pipetting, and then boil up for denaturation for 5 min. Separate the total protein by 10% SDS-PAGE gel, and then transfer it to a PVDF membrane. Block it with 5% BSA at room temperature for 2 h, incubate it with a Flag antibody (abcam) at 4°C overnight, and wash it with TBST for three times. Incubate it with the second antibody at room temperature for 1 h, and wash it with TBST for three times. Stain with an enhanced chemiluminescent (ECL) solution, and detect through imaging by the Tannon imaging system on whether the target band exists.

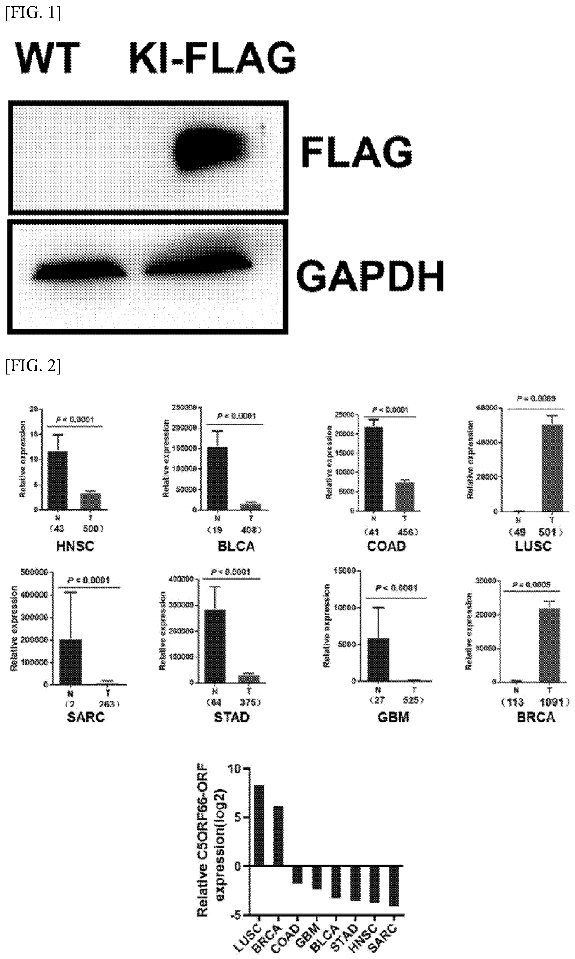

The results are shown in FIG.1. The Western blot assay could detect the Flag band, indicating that C5ORF66-AS1 indeed has a coding ability.

### Embodiment 2

Analysis of the expression levels of MP36 in human tumor tissues and normal tissues.

RNA-seq sequencing files and clinical information for cancer tissues for 32 types of tumors including head and neck cancer, glioma, thyroid cancer, esophageal squamous cell carcinoma, lung cancer, liver cancer, gastric cancer, kidney cancer, breast cancer, ovarian cancer, cervical cancer, bladder cancer, colorectal cancer, pancreatic cancer, osteosarcoma, and skin cancer, and normal tissues were downloaded by the TCGA standard method. Statistical analysis used the R language (version 3.1.1) software. Program packages (heatmap, venndiagram, hist, etc.) needed to be installed and loaded. Then the DESeq and edgeR program packages were used to analyze the expression levels of C5ORF66-AS1 and identify the tumor types with differential expression (as shown in Table 1). Criteria for judging differential expression: (1) | The expression level of cancer/adjacent tissues | is >2, (2) P<0.05.

**Table 1 Analysis of the expression levels of MP36 in human tumor tissues and normal tissues (cancer/adjacent tissues)**

| Tumor Type | Number of Cases | Fold Change of Expression | Value *P* |
|---|---|---|---|
| Lung cancer | 501 | 8.34 | 3.49E-25 |
| Breast cancer | 1091 | 6.15 | 3.50E-28 |
| Colon cancer | 456 | -1.78 | 8.08E-11 |
| Glioma | 525 | -2.36 | 0.034 |
| Bladder cancer | 408 | -3.27 | 1.98E-15 |
| Gastric cancer | 375 | -3.54 | 5.83E-32 |
| Head and neck cancer | 500 | -3.73 | 4.96E-18 |
| Sarcoma | 263 | -4.08 | 0.019 |

As shown in Table 1 and FIG. 2, the expression levels of MP36 in cancer tissues and adjacent tissues were analyzed in comparison with normal tissues. It was found that compared with adjacent tissues, MP36 was significantly overexpressed in breast cancer and lung cancer, and its expression levels were significantly decreased in colon cancer, glioma, bladder cancer, gastric cancer, head and neck cancer, and sarcoma tissues.

### Embodiment 3

The expression levels of MP36 in head and neck cancer patients and normal adjacent tissues were detected by fluorescence quantitative PCR.

### (1) Collection of specimens

With the patient's informed consent, head and neck cancer as well as adjacent tissue specimens were collected during the operation. After being washed with normal saline, they were stored in liquid nitrogen or in a -80°C refrigerator for future use.

### (2) Primer design

Based on the sequence information of MP36, specific primers were designed. The sequences are as follows:
Upstream primer (SEQ ID NO.3): agggcccctccacggaggt
Downstream primer (SEQ ID NO.4): ctccctcggggcgtaggct

### (3) The expression levels of MP36 in head and neck cancer patients and normal adjacent tissues were detected by real-time quantitative PCR.

According to the Trizol manual of TIANGEN BIOTECH, the total RNA of the samples was collected and extracted. Then, the purity and concentration of the extracted RNA was quantified by a NanoDrop ND-1000 nucleic acid quantification instrument. The integrity of the extracted RNA was inspected and ensured by agarose gel electrophoresis. The TaKaRa reagent kit PrimeScripeTM RT reagent Kit with gDNA Eraser (Perfect Real Time) was used to reverse-transcribe the extracted total RNA to synthesize cDNA. The TaKaRa reagent kit SYBR^{®} Premix Ex Taq^{™} II (Tli RNaseH Plus) was used to perform qPCR. The reaction system is shown in Table 2:

**Table 2 PCR system**

| Reagent | Dose (µL) |
|---|---|
| SYBR Premix Ex Taq II (Tli RNaseH Plus) (2×) | 12.5 |
| PCR Forward Primer (10 µM) | 1 |
| PCR Reverse Primer (10 µM) | 1 |
| DNA template (<100 ng) | 2 |
| Sterile water | 8.5 |
| Total | 25 |

After the above components were mixed well, the following procedure proceeded: initial denaturation 95°C 30 s, 40 cycles; 95°C 5 s, 60°C30 s.

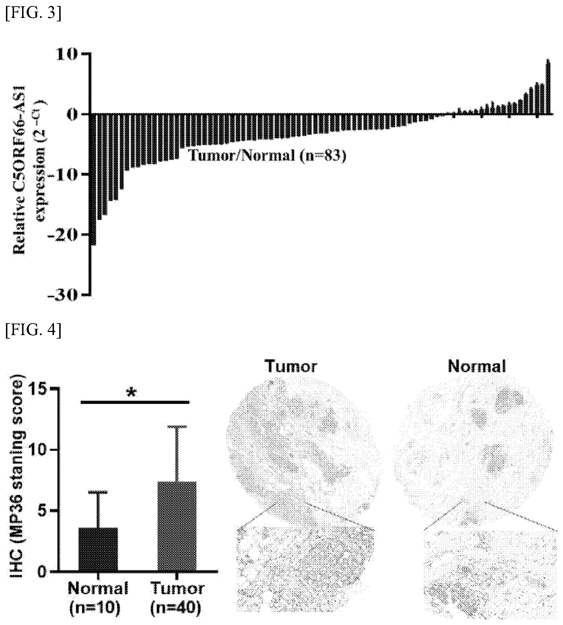

The specificity of the reaction was determined based on the melting curve, and the relative expression level of MP36 was calculated using the formula 2^{-Δ Δ Ct}. The results are shown in FIG. 3. In approximately 75% of the head and neck cancer samples, the expression level of MP36 was significantly lower than that in normal adjacent tissues. The expression trend was similar to that analyzed in the TCGA database, indicating that MP36 can serve as a potential marker for the diagnosis of head and neck cancer.

### Embodiment 4

The expression levels of MP36 in breast cancer patients and normal adjacent tissues were detected by immunohistochemistry.

40 clinical tissue samples of breast cancer and 10 adjacent tissue samples were collected, paraffin chips were prepared for them, and the monoclonal antibody of MP36 was used for immunohistochemical experiments. The specific operation is as follows:
(1) After obtaining the tissue paraffin block, cut it into slices, and bake them at 60°C for 3 h.
(2) Remove the paraffin by immersing them in xylene, xylene, and 50% ethanol each for 10 min in sequence.
(3) Soak them in 100% ethanol, 90% ethanol, 80% ethanol, and 70% ethanol each for 10 min in sequence, and then rinse them in 50% ethanol for 5 min.
(4) Wash them with PBS for 3 times, 10 min per time.
(5) Soak them in 3% hydrogen peroxide at room temperature for 10 min to inactivate the endogenous HPR.
(6) Wash them with PBS for 3 times, 10 min per time.
(7) Perform antigen restoration by heating in a microwave oven, and then reduce the temperature in a water bath to room temperature.
(8) Wash them with PBS for 3 times, 10 min per time, and circle the tissues with an immunohistochemical pen, and dry them for 15 s.
(9) Dropwise add 5% sheep serum blocking solution to block for 70 min.
(10) Incubate the first antibody of MP36 at 4°C overnight, and wash with PBST for three times, 10 min per time.
(11) Incubate the second antibody at room temperature for 1 h, and wash with PBST for three times, 10 min per time.
(12) Stain with DAB for 10 min, and rinse with PBST to terminate the staining.
(13) Re-stain with hematoxylin for 7 min, and then rinse for 5 min.
(14) Dehydrate, seal the slices and observe under a microscope.

The results are shown in Figure 4. They are similar to the analysis conducted using the TCGA database. Compared with the adjacent tissue samples, MP36 was significantly overexpressed in breast cancer, indicating that MP36 can serve as a potential new indicator for the diagnosis of breast cancer.

### Embodiment 5

The effect of overexpression of MP36 on the proliferation ability of human head and neck cancer cells

Overexpress MP36 in head and neck cancer cells CAL27 by using the lentiviral transfection technology (named as MP36-OE), name the control group CAL27 cells as Ctrl, culture the cells of the two groups in a 37°C 5% CO₂ incubator until the cell density reached above 90%, digest and collect the cells with trypsin, resuspend the cells in a culture medium, count the cells under a microscope, adjust the cell concentration to 3.0×10⁴ cells/mL, inoculate the cell suspension into a 96-well plate, 100 µL per well, and incubate the cells in a 37°C 5% CO₂ incubator for 48h. Add 10 µL of CCK8 reagent to each well in a dark environment, keep it in a 37°C water bath away from light for 4 h, start the microplate reader for warmup 30 min in advance, determine the absorbance of each well at 450nm after the incubation, and analyze the data. Independently repeat the experiment for three times, express the results obtained from the experiment with mean±SD, and conduct a statistical T-test. *P<0.05 indicates a significant difference, and **P<0.01 indicates an extremely significant difference.

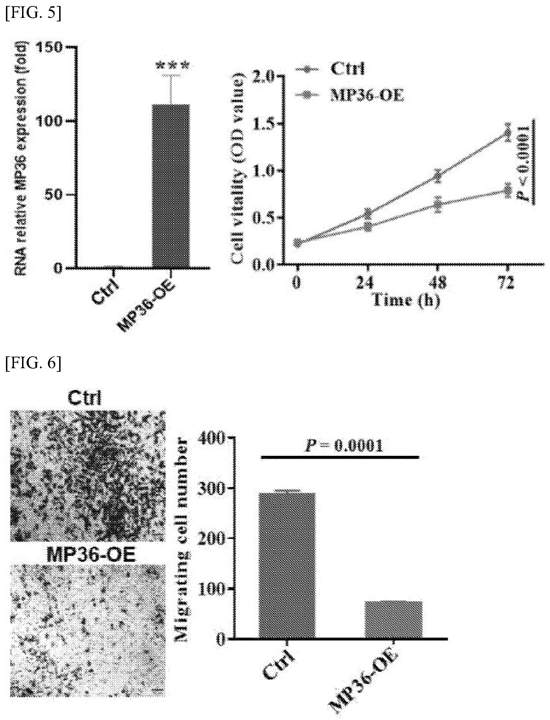

The results are shown in FIG. 5. Compared with the control group, overexpression of MP36 could significantly inhibit the proliferation of head and neck cancer CAL27 cells.

It suggests that the novel micropeptide MP36 can exert an anti-tumor effect by inhibiting the proliferation of head and neck cancer cells.

### Embodiment 6

The effect of overexpression of MP36 on the migration ability of human head and neck cancer cells Inoculate the head and neck cancer cells overexpressing MP36 that were constructed by using the lentiviral transfection technology, and the control cells into a transwell chamber, 100 µL per well. Then, add 0.6 mL of complete medium containing 10% FBS to the lower transwell chamber to stimulate cell migration, and culture at 5% CO₂ 37°C for 48 h. Discard the medium from the wells, fix with methanol at room temperature for 30 min, stain with 0.1% crystal violet at room temperature for 10 min, rinse with clear water, wipe off the cells that did not migrate from the upper layer with a cotton swab, observe under a microscope and select four fields of view for photography and counting. Independently repeat the experiment for three times, express the results obtained from the experiment with mean±SD, and conduct a statistical T-test. *P<0.05 indicates a significant difference, and **P<0.01 indicates an extremely significant difference.

The results are shown in FIG. 6. Compared with the control group, overexpression of MP36 could significantly inhibit the migration of head and neck cancer CAL27 cells.

It suggests that the novel micropeptide MP36 can exert an anti-tumor effect by inhibiting the migration of head and neck cancer cells.

### Embodiment 7

The effect of interfering with MP36 on the proliferation ability of human breast cancer and lung cancer cells

Specific siRNA was designed based on the nucleotide sequence of MP36. The sequence is shown in SEQ ID NO: 5.

Culture breast cancer cells MDA-MB-231 and MCF7 and lung cancer cells A549 in a 37°C 5% CO₂ incubator, digest the cells 24 h in advance, inoculate the cells to a 12-well plate in a density of 8×10⁴ cells/mL, and transfect the cells when the cell confluence reached 60%. Add 50 µL of DMEM medium into a 1.5 mL sterile EP tube, then add 8 µL of transfection reagent, mix them well by pipetting, and let it stand at room temperature for 5 min. Add 50 µL of DMEM medium into other two EP tubes, then add 6 µL of siRNA (si-MP36 and si-NC), respectively, mix them well by pipetting, and let it stand at room temperature for 5 min. Dropwise add the transfection reagent mixture obtained in the first step into the second EP tube, mix it well by pipetting, and incubate it at room temperature for 20 min. Suck out the original culture medium in the 6-well plate and replace it with fresh complete medium during the incubation process. Dropwise add the transfection mixture into the 6-well plate after the incubation, mix it well by shaking, put them in the incubator for culturing. Replace the medium after 6 h, and carry out digestion and conduct a functional experiment after 48 h. The cell proliferation experiment was conducted as described in Embodiment 4, with the groups being si-NC and si-MP36. The experiment was independently repeated for three times, the results obtained from the experiment were expressed as mean±SD, and statistical T-test was conducted. *P<0.05 indicates a significant difference, and ★★P<0.01 indicates an extremely significant difference.

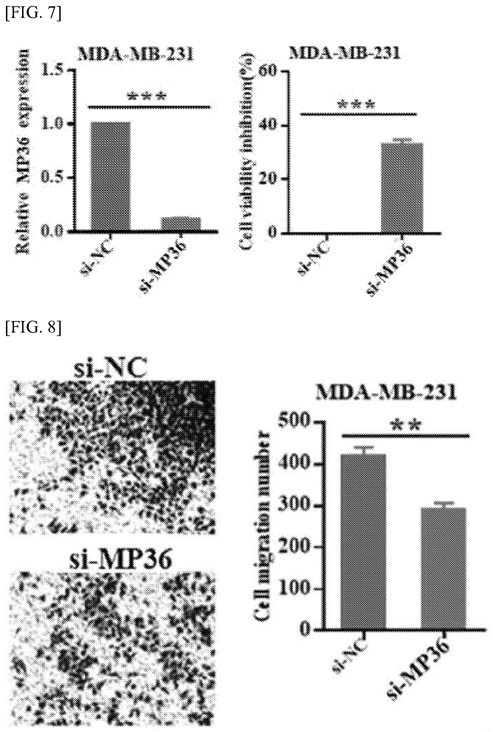

The results are shown in FIG. 7-FIG. 9. Compared with the control group, knocking down MP36 could significantly inhibit the proliferation of breast cancer cells MDA-MB-231 and MCF7 and lung cancer cells A549. This indicates that by targeting and inhibiting the new micropeptide MP36, an anti-tumor effect is exerted by inhibiting the proliferation of breast cancer and lung cancer cells.

### Embodiment 8

The effect of interfering with MP36 on the migration ability of human breast cancer and lung cancer cells

Likewise, inoculate breast cancer cells MDA-MB-231 and MCF7 and lung cancer cells A549 transfected with si-NC and si-MP36 to a transwell chamber, 100 µL per well. Then, add 0.6 mL of complete medium containing 10% FBS to the lower transwell chamber to stimulate cell migration, and culture at 5% CO₂, 37°C for 48 h. Discard the medium from the wells, fix with methanol at room temperature for 30 min, stain with 0.1% crystal violet at room temperature for 10 min, rinse with clear water, wipe off the cells that did not migrate from the upper layer with a cotton swab, observe under a microscope and select four fields of view for photography and counting. Independently repeat the experiment for three times, express the results obtained from the experiment with mean±SD, and conduct a statistical T-test. *P<0.05 indicates a significant difference, and **P<0.01 indicates an extremely significant difference.

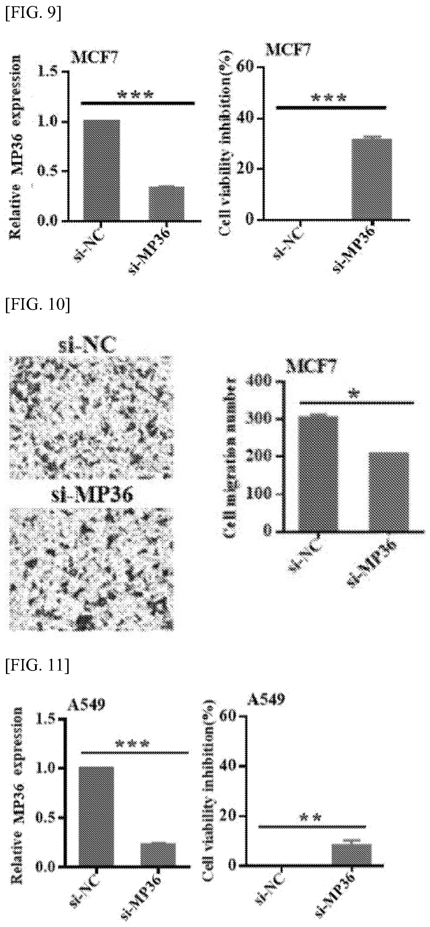

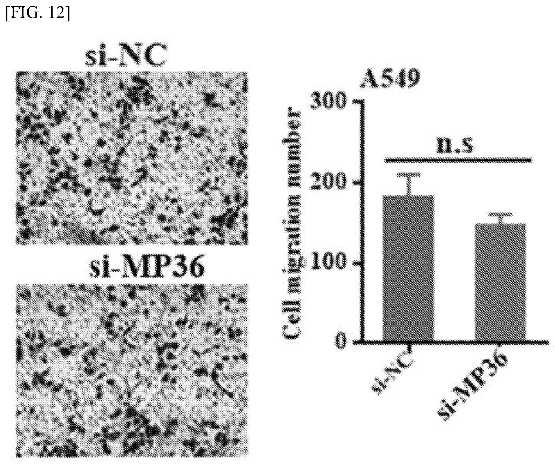

The results are shown in FIG. 10 - FIG. 12. Compared with the control group, knocking down MP36 could significantly inhibit the migration of breast cancer cells MDA-MB-231 and MCF7, but had no effects on the migration of lung cancer cells A549. This indicates that by targeting and inhibiting the new micropeptide MP36, an anti-tumor effect is exerted by inhibiting the migration of breast cancer.

### Embodiment 9

The determination of the mode of action of MP36 through homologous mutations revealed that MP36 functions in the form of micropeptide rather than lncRNA.

In order to prove that it is the micropeptide MP36 rather than the lncRNA C5ORF66-AS1 that plays the functional role, we constructed a lentiviral vector with the same amino acid sequence but altered RNA sequence and changed secondary structure through synonymous mutation, thereby achieving overexpression of the micropeptide MP36 without altering the level of the lncRNA. The cell line of the MP36 synonymous mutation obtained from transfection was named 231-66TY cell line. At the same time, the initiation codon ATG of the MP36 ORF was mutated to ATT by means of lentivirus transfection, so that this sequence could not translate the micropeptide MP36. And the cell line was named 231-66Lnc cell line.

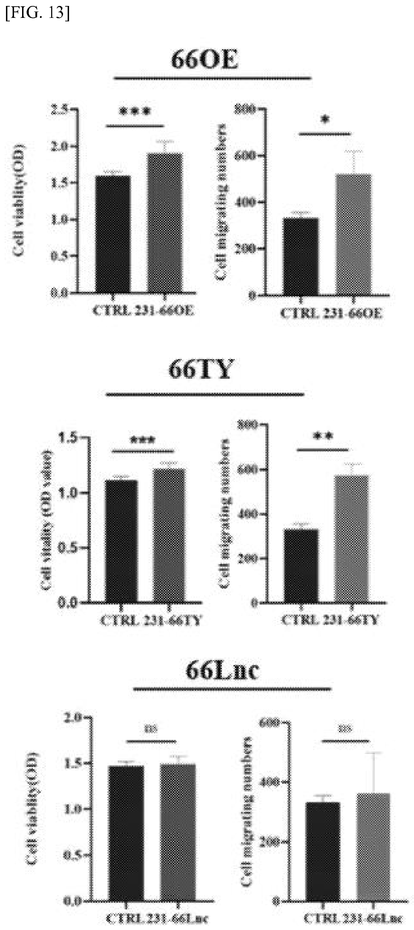

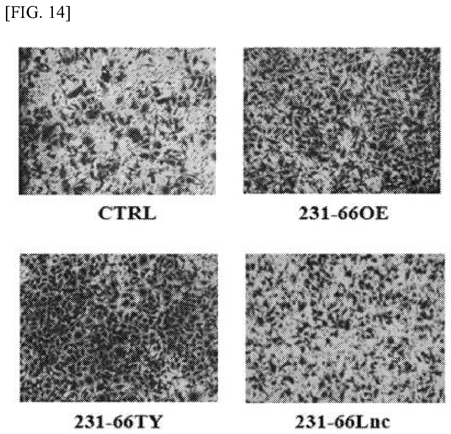

The results are shown in FIG. 13 - FIG. 14. Compared with the control group, the 231-66TY cell line could promote the proliferation and migration of cells, while the 231-66Lnc cell line had no significant effect on the proliferation and migration of cells. This indicates that it is the micropeptide MP36 rather than the lncRNA that plays a functional role.

## Claims

1. A micropeptide, wherein the amino acid sequence of the micropeptide is any of the following ones:
(a) an amino acid sequence shown in SEQ ID NO. 1;
(b) an amino acid sequence that has a homology of more than 85% with the amino acid sequence shown in SEQ ID NO. 1;
(c) an amino acid sequence formed by replacing, deleting or adding one or several amino acid residues in the amino acid sequence shown in SEQ ID NO.1, which has the same function as the original sequence.

2. A nucleotide sequence coding the micropeptide described in claim 1.

3. A recombinant vector containing the nucleotide sequence described in claim 2.

4. A use of the micropeptide described in any one of claims 1-3 in preparing reagents or drugs for detecting, preventing or treating tumors.

5. The use according to claim 4, wherein the tumors are one or more of human lung cancer, breast cancer, colon cancer, glioma, bladder cancer, gastric cancer, head and neck cancer, and sarcoma.

6. The use according to claim 4, wherein the treatment of tumors is achieved by overexpressing the nucleotide sequence shown in SEQ ID NO. 2 in the tumors; or by using the specific small interfering RNA of the nucleotide sequence shown in SEQ ID NO. 2; or by coupling the micropeptide with cell-penetrating peptides.

7. The use according to claim 4, wherein the nucleotide sequence of the specific small interfering RNA is shown in SEQ ID NO: 5.

8. The use according to claim 4, wherein the prevention or treatment of tumors includes inhibiting the growth and/or metastasis of tumor cells.

9. A pharmaceutical composition, wherein the pharmaceutical composition contains the micropeptide described in claim 1, or specific small interfering RNA of the micropeptide, or a molecule that promotes the degradation of the micropeptide and a pharmaceutically acceptable carrier thereof.

10. A tumor detection kit, wherein the kit contains specific primer pairs and/or antibodies designed for the nucleotide sequence described in claim 2; preferably, the nucleotide sequences of the specific primer pairs are shown in SEQ ID NO. 3 and SEQ ID NO. 4.
